# EUROPEAN PATENT SPECIFICATION

(11) **EP 3 582 229 B1**
(45) Date of publication and mention of the grant of the patent: **20.11.2024**
(21) Application number: 19177880.2
(22) Date of filing: 03.06.2019
(51) Int. Cl.: G16H 40/67, A61M 5/168, A61M 5/14, G16H 20/17

(54) **INTRAVENOUS DRIP MONITORING DEVICE, INTRAVENOUS DRIP MONITORING SYSTEM AND METHOD THEREOF**
VORRICHTUNG ZUR ÜBERWACHUNG EINES INTRAVENÖSEN TROPFS, SYSTEM ZUR ÜBERWACHUNG EINES INTRAVENÖSEN TROPFS UND VERFAHREN DAFÜR
DISPOSITIF DE SURVEILLANCE DE GOUTTE-À-GOUTTE INTRAVEINEUX, SYSTÈME DE SURVEILLANCE DE GOUTTE-À-GOUTTE INTRAVEINEUX ET PROCÉDÉ ASSOCIÉ

(30) Priority: 04.06.2018 TW 107119210; 28.11.2018 TW 107142537
(43) Date of publication of application: 18.12.2019
(73) Proprietor: Mikotek Technology Inc., Taipei City 114063 (TW); Yang, Ching-Wen, Taipei City 116 (TW)
(72) Inventor: YANG, Ching-Wen, Wenshan Dist. Taipei City 116 (TW)
(74) Representative: Torggler & Hofmann Patentanwälte - Innsbruck

(56) References cited:
- US-A1- 2015 346 013

## Description

### FIELD OF INVENTION

The present application relates to a real-time monitoring technology. More particularly, the present application relates to an intravenous drip monitoring device, an intravenous drip monitoring system and method thereof.

### DESCRIPTION OF RELATED ART

Medical intravenous drip is driven by gravity mostly, the container is hanging on a stable drip stand and the liquid substance inside the container flows down naturally into intravenous. Currently, the intravenous drip is still monitoring manually, the alarm system is activated manually and required medical staffs handling while the drip ends or abnormal condition happened. An example of an intravenous drip monitoring device according to the prior art is disclosed in US 2015/0346013 A1.

Moreover, because an intravenous drip requires individually setting according to different types and brands of drugs, therefore the drip weight monitoring also need to adjust to meet different kinds of drip. However, it will increase the inconvenience of users. In the lack of efficient monitoring, the burden on medical staff will increase. Therefore, an intravenous drip real-time monitoring system and method is required.

### SUMMARY

An aspect of the disclosure is to provide an intravenous drip monitoring device according to the appended independent claim 1.

Another aspect of the disclosure is to provide an intravenous drip monitoring system (not part of the invention). The intravenous drip monitoring system includes a first server, an intravenous drip monitoring device and an electronic device. The first server is communicatively connected with the intravenous drip monitoring device and the electronic device. The intravenous drip monitoring device is configured for detecting a drip monitoring information, and transmitting the drip monitoring information to the first server. The electronic device is configured for receiving a drip information transmitted from the first server, wherein the first server is configured for receiving the drip monitoring information transmitted from the intravenous drip monitoring device, and comparing the drip monitoring information and a setting information to calculate a time remaining and a flow rate of drip, and the first server is further configured for transmitting a control information to the intravenous drip monitoring device. Wherein the drip information includes the drip monitoring information, the time remaining and the flow rate of drip.

Another aspect of the disclosure is to provide an intravenous drip monitoring method (not part of the invention). The intravenous drip monitoring method includes operations of: detecting a drip monitoring information and transmitting the drip monitoring information to the first server by an intravenous drip monitoring device; comparing the drip monitoring information and a setting information to calculate a time remaining and a flow rate of drip by the first server; transmitting a drip information to an electronic device by the first server, wherein the drip information comprises the drip monitoring information, the time remaining and the flow rate of drip; and transmitting a control information to the intravenous drip monitoring device by the first server.

Intravenous drip real-time monitoring system and method are capable of improving the formerly intravenous drip monitoring system done by manual monitoring, utilizing the weight sensor to detect the weight of drip in real time and sending out an alert sound to caretaker remind him/her replace the drip. The intravenous drip real-time monitoring system will also send the setting information of drip from the cloud server to drip monitoring device. In some embodiments, this disclosure is able to real-time monitoring and convenient setting up the drip monitoring device.

### BRIEF DESCRIPTION OF THE DRAWINGS

Aspects of the present disclosure are best understood from the following detailed description when read with the accompanying figures. It is noted that, in accordance with the standard practice in the industry, various features are not drawn to scale. In fact, the dimensions of the various features may be arbitrarily increased or reduced for clarity of discussion.
FIG. 1 is a functional block diagram illustrating an intravenous drip monitoring system.
FIG. 2A is a functional block diagram illustrating an intravenous drip monitoring device according to an embodiment of the disclosure.
FIG. 2B is a functional block diagram illustrating an intravenous drip monitoring device according to an embodiment of the disclosure.
FIG. 3 is a flow diagram illustrating an intravenous drip monitoring method.
FIG. 4 is a flow diagram illustrating step S310.
FIG. 5 is a flow diagram illustrating step S320.

### DETAILED DESCRIPTION

It will be understood that, in the description herein and throughout the claims that follow, when an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Moreover, "electrically connect" or "connect" can further refer to the interoperation or interaction between two or more elements.

It will be understood that, in the description herein and throughout the claims that follow, although the terms "first," "second," etc. may be used to describe various elements, these elements should not be limited by these terms. These terms are only used to distinguish one element from another. For example, a first element could be termed a second element, and, similarly, a second element could be termed a first element, without departing from the scope of the embodiments.

It will be understood that, in the description herein and throughout the claims that follow, the terms "comprise" or "comprising," "include" or "including," "have" or "having," "contain" or "containing" and the like used herein are to be understood to be open-ended, i.e., to mean including but not limited to.

It will be understood that, in the description herein and throughout the claims that follow, the phrase "and/or" includes any and all combinations of one or more of the associated listed items.

It will be understood that, in the description herein and throughout the claims that follow, words indicating direction used in the description of the following embodiments, such as "above," "below," "left," "right," "front" and "back," are directions as they relate to the accompanying drawings. Therefore, such words indicating direction are used for illustration and do not limit the present disclosure.

It will be understood that, in the description herein and throughout the claims that follow, unless otherwise defined, all terms (including technical and scientific terms) have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

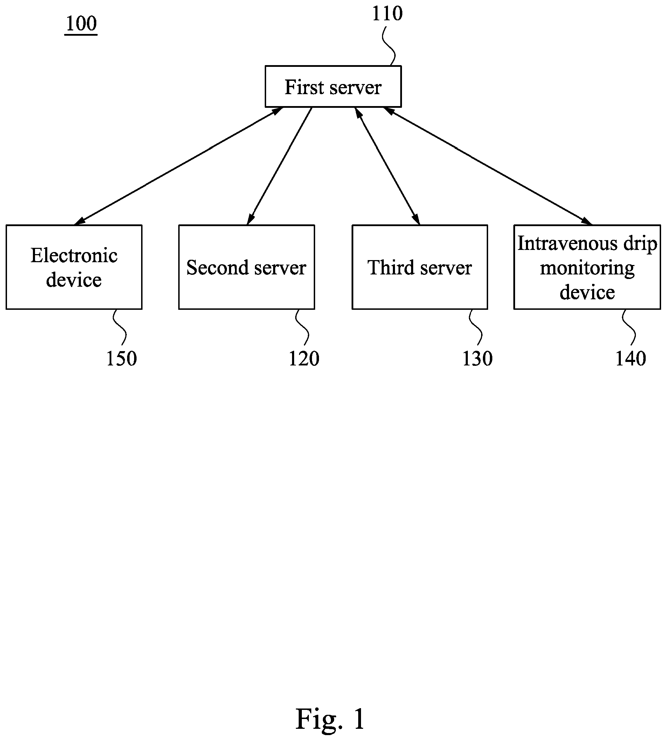

Reference is made to FIG. 1, which is a functional block diagram illustrating an intravenous drip monitoring system 100. As shown in FIG. 1, the intravenous drip monitoring system 100 includes a first server 110, a second server 120, a third server 130, an intravenous drip monitoring device 140 and the electronic device 150. The first server 110 is communicatively connected with the second server 120, the third server 130, the intravenous drip monitoring device 140 and the electronic device 150. The intravenous drip monitoring device 140 is configured to detect a drip monitoring information, and to transmit the drip monitoring information to the first server 110. The first server 110 is configured to transmit a drip information to the second server 120, the third server 130 and the electronic device 150. The third server 130 is configured to transmit a monitoring information to the first server 110. The first server 110 is configured to compare the drip monitoring information and a setting information to calculate a time remaining and a flow rate of drip. The drip information includes the drip monitoring information, the time remaining and the flow rate of drip. The electronic device 150 is configured to transmit a control information to the first server 110, and the first server 110 is configured to transmit the control information to the intravenous drip monitoring device 140.

The second server 120 can be implemented as a server inside the hospital, and the third server 130 can be implemented as a medical cloud server of other business. The electronic device 150 can be implemented as a smart phone, a tablet, or a wearable device used by patients, patient's family or medical staffs.

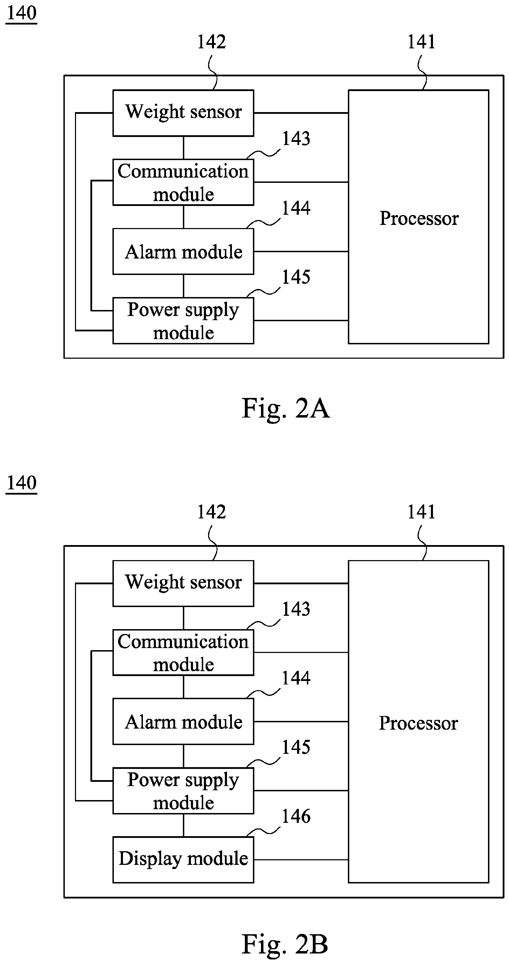

Reference is made to FIG. 2A, which is a functional block diagram illustrating an intravenous drip monitoring device 140 according to an embodiment of the disclosure. As shown in FIG. 2A, the intravenous drip monitoring device 140 includes a processor 141, a weight sensor 142, a communication module 143, an alarm module 144 and a power supply module 145. The processor 141 is electrically connected to the weight sensor 142, the communication module 143, the alarm module 144 and the power supply module 145. The power supply module 145 is electrically connected to the weight sensor 142, the communication module 143 and the alarm module 144. The processor 141 can be connected to the first server 110 by wire. In another embodiment, the processor 141 can be wirelessly transmitted to the first server 110 through the communication module 143.

Afterwards, the weight sensor 142 is configured to detect a weight information of drip, and to transmit the weight information to the processor 141. The communication module 143 is configured to receive a warning value and control information transmitted from the first server 110. The alarm module 144 is configured to generate a warning signal. The power supply module 145 is configured to supply the power source to the processor 141, the weight sensor 142, the communication module 143, and the alarm module 144. The processor 141 is configured to determine whether the weight information is less than or equal to the warning value, if the weight information is less than or equal to the warning value, the processor 141 is configured to enable or disable the alarm module 144 according to the control information.

In the embodiment, the processor 141 can be implemented by a microcontroller, a microprocessor, a digital signal processor, an application specific integrated circuit, a central processing unit, a control circuit and/or a graphics processing unit. The communication module 143 can be implemented by a global system for mobile communication, a personal handy-phone system, a long term evolution, a worldwide interoperability for microwave access, a wireless fidelity, etc. The alarm module 144 can be implemented by a buzzer, a lightemitting device or a combination can generate alert message in the form of sound or flash light. The power supply module 145 can be implemented by battery or the power supply circuit. The electronic device 150 can be implemented by the mobile device, the wearable device or other devices that can receive signals and send out a message.

In another embodiment, reference is made to FIG. 2B, which is a functional block diagram illustrating an intravenous drip monitoring device 140 according to an embodiment of the disclosure. The difference between the embodiment shown in FIG. 2A and the embodiment shown in FIG. 2B is that the intravenous drip monitoring device 140 further includes a display module 146, and the display module 146 is electrically connected to the processor141 and the power supply module 145. The display module 146 is configured to display the drip monitoring information and a setting information. The power supply module 145 is further configured to supply the power source to the display module 146. The display module 146 can be implemented by the display panel or the touch display panel.

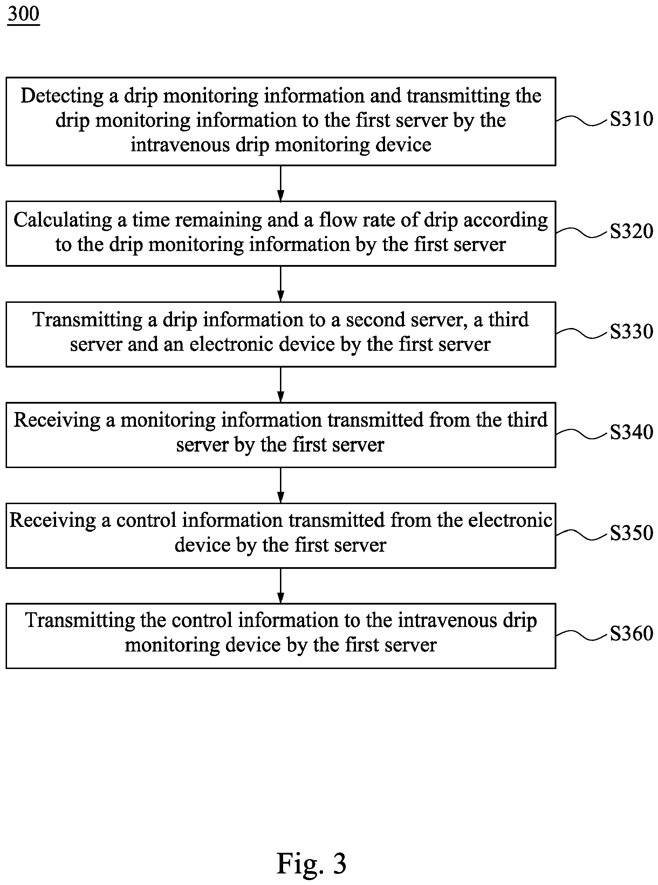

The operation of the intravenous drip monitoring system 100 is described in detail in accompany with FIG. 1, FIG. 2 and FIG. 3. Reference is made to FIG. 3. FIG. 3 is a flow diagram illustrating an intravenous drip monitoring method 300. The intravenous drip monitoring method 300 can be used in the intravenous drip monitoring system 100 shown in FIG. 1 and the intravenous drip monitoring device 140 shown in FIG. 2A and FIG. 2B. The intravenous drip monitoring method 300 includes following steps (The steps are not retired in the sequence in which the steps are performed. That is, unless the sequence of the steps is interchangeable, and all or part of the steps may be simultaneously, partially simultaneously, or sequentially performed).

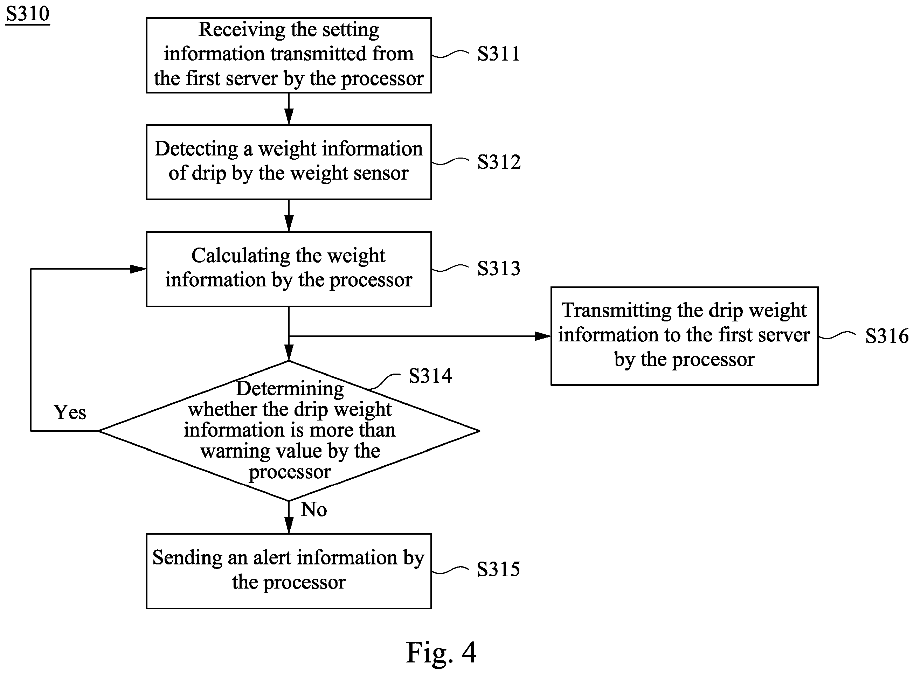

Afterwards, the intravenous drip monitoring method 300 firstly executes step S310 to detect a drip monitoring information and to transmit the drip monitoring information to the first server 110 by the intravenous drip monitoring device 140. The step S310 further includes steps S311-S316, reference is made to FIG. 4, which is a flow diagram illustrating step S310. As shown in FIG. 4, the intravenous drip monitoring method 300 firstly executes step S311 to receive the setting information transmitted from the first server 110 by the processor 141. The first server 110 store the setting information of different drips, the setting information includes an empty drip bag weight, the warning value, a drip initial weight, a medicine type, a medicine brand, and a burette type, or other information recorded in the medical advice. When users install the intravenous drip monitoring system 100 on drip, they may utilize the processor 141 to download the corresponding drip setting information from the first server 110 via the internet to control the weight sensor 142 according to the setting information.

Afterwards, the intravenous drip monitoring method 300 executes step S312 to detect a weight information of drip by the weight sensor 142 and step S313 to calculate the weight information by the processor 141. When the drip is installed the intravenous drip monitoring system 100, the weight sensor 142 is configured to detect the weight of drip, and transmit the weight information to the processor 141. The processor 141 is configured to calculate the weight information from the weight sensor 142 continuously.

Afterwards, the intravenous drip monitoring method 300 executes step S314 and step S315 to determine whether the weight information is more than the warning value by the processor 141, if not, sending an alert information by the processor 141. The processor 141 is configured to detect whether the drip weight is less than or equal to the warning value in the setting information continuously, once the weight information is less than or equal to the warning value, the processor 141 is configured to send the alert information to the alarm module 144.

Afterwards, the intravenous drip monitoring method 300 executes step S316 to transmit the weight information to the first server 110 by the processor 141. The processor 141 is configured to transmit the drip monitoring information to the first server 110 continuously, the drip monitoring information includes the drip weight, the condition of the alarm module 144 (e.g. monitoring condition or alert condition), the identification number of drip, the battery level of the processor 141, etc., and the first server 110 is configured to store the drip monitoring information in the memory.

After replacing the drip by the caregiver, the intravenous drip monitoring device 140 is configured to automatically detect and determine whether the weight information is same as previous weight information, if the weight information is same as the previous weight information, it can be realized that the setting information of the replaced drip is the same as the previous one, so there is no need to set the additional warning value. However, if the weight information is different from the previous weight information, it can be realized that the setting information of the replaced drip is different from the previous one, so it is need to enter another warning value. Alternatively, the caregiver can reset the warning value, if the drip with different setting information is replaced and the warning value is need to adjust.

Afterwards, the processor 141 is configured to transmit the drip monitoring information to the first server 110. The drip monitoring information includes an identifier of the drip monitoring device, a name of the drip monitoring device, a status of the drip monitoring device, the warning value and the weight information. The name of the drip monitoring device can be set to the name of user, such as father's drip. The status of the drip monitoring device can be realized as current status of the intravenous drip monitoring device 140, for example, monitoring, warning, suspending, waiting, emptying, etc. The weight information is detected by the weight sensor 142, for the sake of brevity, those descriptions will not be repeated here.

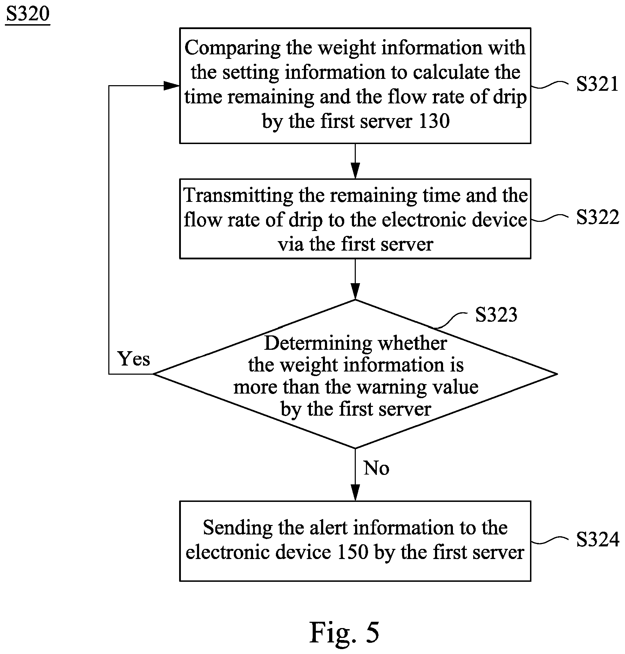

Afterwards, the intravenous drip monitoring method 300 firstly executes step S320 to calculate a time remaining and a flow rate of drip according to the drip monitoring information by the first server 110. The step S320 further includes steps S321-S324, reference is made to FIG. 5, which is a flow diagram illustrating step S320. As shown in FIG. 5, the intravenous drip monitoring method 300 further executes step S321 to compare the weight information with the setting information to calculate the time remaining and the flow rate of drip by the first server 110. The processor 141 is configured to transmit the weight information to the first server 110 real-time when the weight information is received. Then the first server 110 is configured to utilize the setting information to calculate the time remaining of drip. By comparing the initial drip weight and the real-time drip weight be able to calculate the remaining time of dripping. For example, the initial drip weight is 500g, after a minute, the remaining weight is 490g, and then the first server 110 can estimate the remaining time of dripping is 50 minutes.

Afterwards, the first server 110 is further configured to calculate the flow rate of drip according to the weight information. For example, the first server 110 can calculate the flow rate (weight information/ time) during a time period, wherein the time period can be set as 20 seconds or 30 seconds, however, the disclosure is not limited thereto. From the above, the flow rate of drip and the time remaining of drip are the information calculated by the first server 110. The drip information includes the drip monitoring information obtained by the intravenous drip monitoring device 140, the time remaining and the flow rate of drip.

Afterwards, the intravenous drip monitoring method 300 executes step S322 to transmit the remaining time and the flow rate of drip to the electronic device 150 via the first server 110. After calculate the remaining time, the first server 110 is configured to transmit the remaining time (50 minutes) to the electronic device 150, and therefore, the holder of electronic device may aware the remaining time of dripping. The electronic device 150 is installed reminding mechanism, for example, the electronic device 150 is configured to send alert information to remind the user (in this state may refer to nurse, caregiver or caretaker) to replace patient's drip.

Afterwards, the intravenous drip monitoring method 300 executes step S323 to determine whether the weight information is more than the warning value by the first server 110 and step S324 if the weight information is less than or equal to the warning value, sending the alert information to the electronic device 150 by the first server 110. The first server 110 is configured to detect whether the weight information is less than or equal to the warning value, once the drip weight is less than or equal to the warning value, the first server 110 is configured to transmit the remaining time of drip and the alert information to the electronic device 150. The first server 110 is configured to transmit the remaining time of drip and alert information to the corresponding electronic device 150 according to the identification number of drip included in the drip data. The alert information can be realized as reminding the caretaker replaces the patient's drip according to the alarm module condition.

When the electronic device 150 is not connected to the first server 110, the electronic device 150 is configured to calculate the remaining time continuously. For example, when the electronic device 150 hold by the caregiver is not communicated with the internet, the electronic device 150 is not connected to the first server 110 real-time. Therefore, the electronic device 150 could not receive the remaining time transmitted by the first server 110. The electronic device 150 will activate the countdown mechanism and calculate the remaining time continuously, until the electronic device 150 is connected with the first server 110. The electronic device 150 will adjust the remaining time according to the remaining time the first server 110 calculated.

The processor 141 is further configured to detect electricity of the power supply module 145 and to transmit an electricity information to the first server 110 via the communication module 143. For example, when the power supply module 145 is implemented by battery, the processor can be utilized to detect the remaining electricity of the battery and to transmit the electricity information and the drip information to the first server 110. The electricity information is used to remind the patient or the caregiver the battery status of the intravenous drip monitoring device 140, so that the patient or the caregiver can be reminded to replace the battery before the battery runs out.

The first server 110 is further configured to record the weight information during a time period, and calculate a weight difference between the current weight information and the previous weight information to determine whether the weight difference is more than a threshold. For example, the initial drip weight is 500g, after three minutes, the remaining weight is 480g, and after five minutes, the remaining weight is 10g. At this time, the processor 141 detects that the weight difference of the drip is -470g, and the weight difference is more than the threshold, so the first server 110 determines that an abnormal condition is happened. The abnormal condition can be realized that someone pulls the intravenous drip monitoring device 140 to cause the weight difference, or the drip may be blocked so that the weight of drip does not change during the time period. The first server 110 can be utilized to detect whether the abnormal condition is still occurred (the weight difference exceed the threshold), if the abnormal condition is relieved, the first server 110 is configured to inform the processor 141 to disable the alarm module 144. However, the abnormal condition is not relieved during the time period, the first server 110 is configured to inform the processor 141 to enable the alarm module 144, and to remind the caregiver that the intravenous drip monitoring device 140 is happened the abnormal condition.

Afterwards, the intravenous drip monitoring method 300 further executes step S330 to transmit the drip information to a second server 120, a third server 130 and an electronic device 150 by the first server 110 and the step S340 to receive a monitoring information transmitted from the third server 130 by the first server 110. The first server 110 is configured to transmitted the drip information to the second server 120, the third server 130 and the electronic device 150 after receiving the drip information. The third server 130 is configured to transmit the monitoring information to the first server 110, wherein the monitoring information can be realized as the physiological information such as blood pressure information, heartbeat information, heart rate information and body temperature information, etc. For example, the first server 110 and the third server 130 can transmit information to each other. The third server 130 can be implemented by a cloud server that collects information such as heartbeat, blood pressure or body temperature, and then the third server 130 can exchange the data with the first server 110.

Alternatively, the first server 110 is configured to transmit the drip information to the third server 130, and the third server 130 can use the drip information for subsequent operations. For example, the drip information can be provided to the user severed by the third server 130. The second server 120 can be implemented as a server inside the hospital. The second server 120 may not be able to share the data with patient personal information to the first server 110, and thus the second server 120 only received the drip information transmitted from the first server 110.

Alternatively, the first server 110 can transmit the drip information with the electricity information to the electronic device 150 to remind the patient or the caregiver the current battery status of the intravenous drip monitoring device 140.

In addition to receiving the drip monitoring information from the processor 141, the first server 110 can also receive the setting information of the drip. For example, the setting information of drip may change due to the update of the capacity of drugs, replace the company, or the drug certificate is expired, and therefore the drip setting can be updated by the user interface (not shown in figure) of the first server 110.

The first server 110 can be utilized to record the patient's drip data. For example, even the disease is identical, the amount of drip may be different due to age, gender or race, such as a 20-30 years old white male and a 0-5 years old Asian female may need different amount of drip. The first server 110 can be utilized to collect the unrecognized personal data and provide the information to medical institution or medical college for researching.

Afterwards, the intravenous drip monitoring method 300 further executes step S350 to receiving the control information transmitted from the electronic device 150 by the first server 110 and the step S360 to transmit the control information to the intravenous drip monitoring device 140 by the first server 110. The electronic device 150 is configured to transmit control information to the first server 110, and the first server 110 is configured to transmit the control information to the intravenous drip monitoring device 140, and then the intravenous drip monitoring device 140 is configured to enable or disable the alarm module 144 according to the control information. For example, the first server 110 can open the authority to the caregiver, and the caregiver can transmit the control information to the first server 110 by using the electronic device 150, thereby controlling the intravenous drip monitoring device 140 to enable or disable the alarm module 144 at a specific time. Therefore, the burden on the caregiver setting the intravenous drip monitoring device 140 at one time can be reduced by controlling multiple of the intravenous drip monitoring device 140.

Afterwards, for example, the caregiver can use the electronic device 150 to set the rest time from 0 am to 6 am, and then the alarm module 144 is in the disable status. When the weight information is less than or equal to the warning value, the alarm module 144 is not generate the alert sound or emit the alert light to remind the caregiver. However, the intravenous drip monitoring device 140 will still send the alert information to the electronic device 150 to remind the caregiver to check the drip.

Afterwards, there is an identification code on the intravenous drip monitoring device 140, and the electronic device 150 is configured to check the drip information and the electricity information according to the identification code. The identification code can be implemented by the one-dimensional barcode or QR code, however, the disclosure is not limited thereto. For example, the patient or the caregiver can directly use the electronic device 150 to scan the identification code and check the drip information and the electricity information of the intravenous drip monitoring device 140, and then they can know the status (the time remaining of drip, the flow rate of drip and the current weight information, etc) of the intravenous drip monitoring device 140. The caregiver may have higher authority to modify the drip information of the intravenous drip monitoring device 140, such as modifying the warning value.

Afterwards, when the electronic device 150 has used the identification code to check the drip information and the electricity information, the first server 110 is configured to record the identification information of the electronic device 150 and the identification number of the checked intravenous drip monitoring device 140. The first server 110 automatically transmits the drip information and the electricity information of the intravenous drip monitoring device 140 to the electronic device 150, and there is no need to rescan the identification code. For example, it is assumed that the caregiver need to care three patients, and the intravenous drip monitoring device 140 of three patients are the intravenous drip monitoring device A, the intravenous drip monitoring device B and the intravenous drip monitoring device C, respectively. When caregiver's electronic device 150 scans the identification code corresponding to the intravenous drip monitoring device A, the intravenous drip monitoring device B and the intravenous drip monitoring device C, respectively, caregiver's electronic device 150 can automatically receive the drip information and the electricity information corresponding to the intravenous drip monitoring device A, the intravenous drip monitoring device B and the intravenous drip monitoring device C.

The intravenous drip monitoring system and method are capable of improving the formerly intravenous drip monitoring system done by manual monitoring, utilizing the intravenous drip monitoring device to detect the weight of drip in real time and sending out an alert sound to caretaker remind him/her replace the drip when the drip weight is less than or equal to the threshold. The intravenous drip monitoring system will also send the setting information to the first server and the first server transforms the drip weight to remaining time, and then it transmits the remaining time to the caregiver's electronic device to remind him/her to replace the drip in time. The setting information of drip transmitted from the first server to the intravenous drip real-time monitoring system. Therefore, the disclosure is able to achieve drip monitoring real-time and convenient setting up the drip monitoring device.

## Claims

1. An intravenous drip monitoring device, comprising:
a processor (141);
a weight sensor (142) electrically connected to the processor (141), configured for detecting a weight information of drip, and transmitting the weight information to the processor (141);
a communication module (143) electrically connected to the processor (141), configured for transmitting a drip monitoring information to a first server (110);
an alarm module (144) electrically connected to the processor (141), configured for generating a warning signal;
a power supply module (145) electrically connected to the processor (141), the weight sensor (142), the communication module (143), and the alarm module (144), configured for supplying power source to the processor (141), the weight sensor (142), the communication module (143), and the alarm module (144);
wherein the processor (141) is configured for determining whether the weight information is less than or equal to a warning value, if the weight information is less than or equal to the warning value, controlling the alarm module (144) to generate the warning signal, **characterized in that** the communication module (143) is configured for receiving the warning value and a control information transmitted from the server (110) and the processor (141) is configured for enabling or disabling the alarm module (144) according to the control information.

2. The intravenous drip monitoring device of claim 1, further comprising:
a display module (146) electrically connected to the processor (141) and the power supply module (145), configured for displaying the drip monitoring information and a setting information.

3. The intravenous drip monitoring device of any of claims 1 to 2, wherein the power supply module (145) is implemented by battery, and the processor (141) is further configured for detecting a remaining electricity of the power supply module (145) and transmitting the remaining electricity information to the first server (110) via the communication module (143).

4. The intravenous drip monitoring device of claim 2, wherein the communication module (143) is configured for receiving the setting information transmitted from the first server (110), wherein the setting information comprises an empty drip bag weight, a drip initial weight, a medicine type, a medicine brand, and a burette type.

## Patentansprüche

1. Eine Vorrichtung zur Überwachung eines intravenösen Tropfs, umfassend:
einen Prozessor (141);
einen Gewichtssensor (142), der elektrisch mit dem Prozessor (141) verbunden und derart konfiguriert ist, dass er eine Gewichtsinformation des Tropfs erfasst und die Gewichtsinformation an den Prozessor (141) sendet;
ein Kommunikationsmodul (143), das elektrisch mit dem Prozessor (141) verbunden und derart konfiguriert ist, dass es eine Information zur Überwachung des Tropfs an einen ersten Server (110) sendet;
ein Alarmmodul (144), das elektrisch mit dem Prozessor (141) verbunden ist und derart konfiguriert ist, um ein Warnsignal zu generieren;
ein Stromversorgungsmodul (145), das elektrisch mit dem Prozessor (141), dem Gewichtssensor (142), dem Kommunikationsmodul (143) und dem Alarmmodul (144) verbunden ist und derart konfiguriert ist, dass es dem Prozessor (141), dem Gewichtssensor (142), dem Kommunikationsmodul (143) und dem Alarmmodul (144) eine Stromquelle zuführt;
wobei der Prozessor (141) derart konfiguriert ist, zu bestimmen, ob die Gewichtsinformation kleiner oder gleich einem Warnwert ist, und wenn die Gewichtsinformation kleiner oder gleich dem Warnwert ist, das Alarmmodul (144) zu steuern, um das Warnsignal zu generieren, **dadurch gekennzeichnet, dass** das Kommunikationsmodul (143) derart konfiguriert ist, dass es den Warnwert und eine vom Server (110) gesendete Steuerungsinformation empfängt, und der Prozessor (141) konfiguriert ist, um das Alarmmodul (144) gemäß der Steuerungsinformation zu aktivieren oder zu deaktivieren.

2. Die Vorrichtung zur Überwachung eines intravenösen Tropfs nach Anspruch 1, ferner umfassend:
ein Display-Modul (146), das elektrisch mit dem Prozessor (141) und dem Stromversorgungsmodul (145) verbunden ist und derart konfiguriert ist, dass es die Informationen zur Überwachung des Tropfs und eine Einstellinformation anzeigt.

3. Die Vorrichtung zur Überwachung eines intravenösen Tropfs nach einem der Ansprüche 1 bis 2, wobei das Stromversorgungsmodul (145) durch eine Batterie implementiert ist, und der Prozessor (141) ferner derart konfiguriert ist, um eine verbleibende Elektrizität des Stromversorgungsmoduls (145) zu erfassen und die Information über die verbleibende Elektrizität über das Kommunikationsmodul (143) an den ersten Server (110) zu senden.

4. Die Vorrichtung zur Überwachung eines intravenösen Tropfs nach Anspruch 2, wobei das Kommunikationsmodul (143) derart konfiguriert ist, dass es die von dem ersten Server (110) gesendeten Einstellinformationen empfängt, wobei die Einstellinformationen ein Leergewicht des Tropfbeutels, ein Anfangsgewicht des Tropfs, einen Medikamententyp, eine Medikamentenmarke und einen Büretten-Typ umfassen.

## Revendications

1. Dispositif de surveillance de goutte-à-goutte intraveineux, comprenant :
un processeur (141) ;
un capteur de poids (142) connecté électriquement au processeur (141), configuré pour détecter une information de poids de goutte-à-goutte, et transmettre l'information de poids au processeur (141) ;
un module de communication (143) connecté électriquement au processeur (141), configuré pour transmettre une information de surveillance de goutte-à-goutte à un premier serveur (110) ;
un module d'alarme (144) connecté électriquement au processeur (141), configuré pour générer un signal d'avertissement ;
un module d'alimentation électrique (145) connecté électriquement au processeur (141), au capteur de poids (142), au module de communication (143), et au module d'alarme (144), configuré pour amener une source d'alimentation électrique au processeur (141), au capteur de poids (142), au module de communication (143), et au module d'alarme (144) ;
dans lequel le processeur (141) est configuré pour déterminer si l'information de poids est inférieure ou égale à une valeur d'avertissement, si l'information de poids est inférieure ou égale à la valeur d'avertissement, commander le module d'alarme (144) pour générer le signal d'avertissement, **caractérisé en ce que** le module de communication (143) est configuré pour recevoir la valeur d'avertissement et une information de commande transmise à partir du serveur (110) et le processeur (141) est configuré pour activer ou désactiver le module d'alarme (144) en fonction de l'information de commande.

2. Dispositif de surveillance de goutte-à-goutte intraveineux selon la revendication 1, comprenant en outre :
un module d'affichage (146) connecté électriquement au processeur (141) et au module d'alimentation électrique (145), configuré pour afficher l'information de surveillance de goutte-à-goutte et une information de paramétrage.

3. Dispositif de surveillance de goutte-à-goutte intraveineux selon l'une quelconque des revendications 1 à 2, dans lequel le module d'alimentation électrique (145) est mis en œuvre par batterie, et le processeur (141) est en outre configuré pour détecter une électricité restante du module d'alimentation électrique (145) et transmettre l'information d'électricité restante au premier serveur (110) par le biais du module de communication (143).

4. Dispositif de surveillance de goutte-à-goutte intraveineux selon la revendication 2, dans lequel le module de communication (143) est configuré pour recevoir l'information de paramétrage transmise à partir du premier serveur (110), dans lequel l'information de paramétrage comprend un poids de sachet de goutte-à-goutte vide, un poids initial de goutte-à-goutte, un type de médicament, une marque de médicament et un type de burette.
